# EUROPÄISCHE PATENTANMELDUNG

(11) **EP 2 036 589 A1**
(43) Veröffentlichungstag der Anmeldung: **18.03.2009**
(21) Anmeldenummer: 08160975.2
(22) Anmeldetag: 23.07.2008
(51) Int. Cl.: A61N 1/375, A61N 1/378

(54) **Batteriebetriebenes Gerät, insbesondere implantierbares medizinelektronisches Gerät**

(30) Priorität: 13.09.2007 DE 102007043660
(71) Anmelder: BIOTRONIK CRM Patent AG, 6341 Baar (CH)
(72) Erfinder: Traulsen, Tim, 01796 Pirna (DE); Drews, Jürgen, 01796 Pirna (DE); Hucke, Thomas, 01217 Dresden (DE)
(74) Vertreter: Lindner-Vogt, Karin L.

(57) **Zusammenfassung**

Batteriebetriebenes Gerät, mit einer elektrisch betriebenen Funktionseinheit und einer elektrochemischen Spannungsquelle, die zusammen in einem im wesentlichen gasdichten Gerätegehäuse untergebracht sind, wobei die elektrochemische Spannungsquelle einen Elektrolyten auf Basis einer ionischen Flüssigkeit und ein beschichtungsfreies Kunststoff-Batteriegehäuse aufweist.

## Beschreibung

Die Erfindung betrifft ein batteriebetriebenes Gerät mit einer elektrisch betriebenen Funktionseinheit und einer elektrochemischen Spannungsquelle, die zusammen in einem im wesentlichen gasdichten Gerätegehäuse untergebracht sind, insbesondere ein implantierbares medizinelektronisches Gerät mit einem solchen Grundaufbau.

Derartige Geräte, speziell auch implantierbare medizinelektronische Geräte, wie Herzschrittmacher, implantierbare Defibrillatoren, Neurostimulatoren oder implantierbare Insulin- oder Medikamentenpumpen etc., sind in großer konstruktiver Vielfalt bekannt. Als Energiequelle enthalten sie heute zumeist ein Lithium-Ionen-Sekundärelement (nachfolgend auch kurz bezeichnet als "Lithiumbatterie"), welches ein gasdicht verschweißtes Metallgehäuse, in der Regel aus Edelstahl oder Titan, aufweist.

Bei solchen Batterien ist dieses aufwendige Gehäuse ein wesentlicher Kostenfaktor, weshalb man unter Kostenaspekten seit längerem nach einer einfacheren Lösung sucht. Grundsätzlich sind hierfür, wegen der leichten Herstellbarkeit und außerordentlich niedrigen Kosten, Kunststoffgehäuse in Betracht zu ziehen.

Der Einsatz von Batterien - speziell Lithiumbatterien - mit Gehäusen aus Kunststoff ist bislang für medizinische Implantate nicht möglich, da in Lithiumbatterien in der Regel flüssige oder gelförmige Elektrolyte auf Basis organischer Lösemittel oder Lösemittelgemische verwendet werden. Bevorzugt verwendet werden organische Carbonate, Ether oder Ester. Organische Polymere sind - vor allem bei erhöhten Temperaturen - durchlässig für Dämpfe dieser Lösemittel, so dass bei Verwendung eines Kunststoffgehäuses in einem gasdicht verschlossenen Implantat Lösemittel in den Innenraum des Implantats gelangen können. Diese Lösemittel können mit anderen Komponenten des Implantats z.B. elektronischen Bauteilen oder Isolationen, reagieren und Störungen oder Defekte auslösen.

Es ist Aufgabe der Erfindung, ein Gerät der eingangs charakterisierten Art bereitzustellen, welches mit einer geeigneten Kombination von Konstruktionsmerkmalen und Materialaspekten dieses grundsätzliche Hindernis umgeht und hierdurch kostengünstiger herstellbar ist.

Diese Aufgabe wird durch ein Gerät mit den Merkmalen des Anspruchs 1 gelöst. Zweckmäßige Fortbildungen des Erfindungsgedankens sind Gegenstand der abhängigen Ansprüche.

Die Erfindung schließt den grundlegenden Gedanken ein, in der elektrochemischen Spannungsquelle des Gerätes einen Elektrolyten auf Basis einer ionischen Flüssigkeit einzusetzen.

Ionische Flüssigkeiten sind Flüssigkeiten, die ausschließlich Ionen enthalten. Es handelt sich also um flüssige Salze, ohne dass das Salz dabei in einem Lösungsmittel gelöst ist. Lange Zeit waren heiße Salzschmelzen (bei Kochsalz über 800°C) die einzigen bekannten Beispiele für derartige Flüssigkeiten. Heutzutage spricht man von ionischen Flüssigkeiten vorrangig im Zusammenhang mit Salzen, die bereits bei Temperaturen unter 100°C flüssig sind.

Beispiele für verwendete Kationen sind alkylierte Imadazolium-, Pyridinium-, Ammoniumoder Phosphonium-Ionen. Als Anionen werden unterschiedlichste Ionen vom einfachen Halogenid über komplexere anorganische Ionen wie Tetrafluoroborate bis hin zu großen organischen Ionen wie Trifluoromethansulfonimid herangezogen. Die Größe der beteiligten Ionen behindert die Bildung eines starken Kristallgitters. Bereits geringe thermische Energie genügt daher, um die Gitterenergie zu überwinden und die feste Kristallstruktur aufzubrechen.

Ionische Flüssigkeiten zeichnen sich durch eine Reihe interessanter Eigenschaften aus: Sie sind thermisch stabil, nicht entzündlich, haben einen äußerst geringen, kaum messbaren Dampfdruck und verfügen über sehr gute Lösungseigenschaften für polare Substanzen und Salze. Darüber hinaus besitzen diese aufgrund ihres rein ionischen Aufbaus auch interessante elektrochemische Eigenschaften, wie z.B. elektrische Leitfähigkeit, die oft auch von einer hohen elektrochemischen Stabilität (d.h. gegen Oxidationen und Reduktionen) begleitet wird. Durch Variation der Seitenketten des Kations und die Auswahl geeigneter Anionen lässt sich z.B. die Löslichkeit in Wasser oder organischen Lösungsmitteln weitgehend frei bestimmen.

Die ersten Publikationen über dessen Einsatz als Katalysatoren (Boon, J.A.; Levisky, J.A.; Pflug, J.L.; Wilkes, J.S.; J. Org. Chemistry, 1986, 51, 480-483) und als Lösungsmittel (Fry, S.E.; Pienta, J.N.; J. Am. Chem. Society, 1985, 107, 6399-6400) für organische Reaktionen gab es Ende der achtziger Jahre. In jüngerer Zeit sind auch Vorschläge zum Einsatz ionischer Flüssigkeiten als neuartige Elektrolyten in elektrochemischen Geräten, wie etwa Lithium-Sekundärelementen, Doppelschicht-Kondensatoren, Brennstoffzellen etc., bekannt geworden; vgl. dazu H. Tokuda et al: "Physicochemical Properties and Structures of Room Temperature lonic Liquids. 1. Variation of Anionic Species", J. Phys. Chem. B 2004, 108, 16593, sowie weitere Literaturhinweise in jener Veröffentlichung.

In ionischen Flüssigkeiten lassen sich Lithium-Salze lösen. Diese Lösungen können als Elektrolyte in Lithiumbatterien eingesetzt werden. Elektrolyte auf Basis Ionischer Flüssigkeiten haben keinen Dampfdruck und damit auch keine Permeabilität durch eine Vielzahl von polymeren Substanzen, d.h. der Elektrolyt kann ein Batteriegehäuse aus Kunststoff nicht durchdringen.

Die Erfindung, speziell also die Verwendung von Kunststoff-Batteriegehäusen (mit anderen Worten: Batteriegehäusen aus organischen Polymeren), hat folgende Vorteile:
- geringes Gewicht
- einfache Verarbeitung
- geringe Kosten
- vielfältige Möglichkeiten bei der Formgebung
- große Auswahl an Materialien.

Wenn nachfolgend von einem Kunststoff-Batteriegehäuse gesprochen wird, so schließt dies ausdrücklich den Einsatz von Composit-Werkstoffen, z.B. Kunststoff/Keramik-Compositen, oder andere Gehäusevarianten ein, deren Hauptbestandteil Kunststoff oder ein solches Compositmaterial ist.

Nach Obigem ist bei der Erfindung insbesondere vorgesehen, dass der Elektrolyt in einem Grade frei von Lösungsmitteln und das Kunststoffgehäuse derart ausgebildet ist, dass bei Betriebstemperatur kein messbares Ausgasen aus dem Batteriegehäuse auftritt. Dies ermöglicht insbesondere eine Realisierung des Gerätes als implantierbares medizinelektronisches Gerät, insbesondere Herzschrittmacher, Defibrillator, Insulin- oder Medikamentenpumpe oder dergleichen, mit gasdicht verschweißtem oder versiegeltem Metall-Gerätegehäuse.

Durch das gasdichte Außengehäuse ist der Zutritt von Wasserdampf in das Geräteinnere und somit auch in das Innere des elektrochemischen Elementes (durch das wasserdampfdurchlässige Kunststoff-Batteriegehäuse) ausgeschlossen. Hiermit ist eine Schädigung von wasserempfindlichen Komponenten etwa einer Lithiumbatterie durch hinzutretenden Wasserdampf sicher unterbunden. Dies ermöglicht es, auf eine zusätzliche Beschichtung des Kunststoffgehäuses zur Erzielung einer die Batteriekomponenten umgebenden Wasserdampfsperre in kostensparender Weise zu verzichten.

In kostengünstigen Realisierungen der Erfindung mit etablierten Großserientechnologien der Kunststoffverarbeitung ist vorgesehen, dass das Batteriegehäuse ein Spritzgussteil oder ein Folien-Tiefziehteil aufweist.

Gemäß einer ersten Variante ist vorgesehen, dass das elektrochemische Element einen flüssigen Elektrolyten und ein flüssigkeitsdicht ausgebildetes Batteriegehäuse, insbesondere mit aufgeschweißtem Deckel, aufweist. Eine hierzu alternative Ausführungsform sieht vor, dass das elektrochemische Element einen gelförmigen Elektrolyten und ein nicht flüssigkeitsdicht ausgebildetes Batteriegehäuse, insbesondere mit einfach formschlüssig eingesetztem Deckel, aufweist. Als Materialien für das Batteriegehäuse sind kostengünstige Massenkunststoffe wie Polyethylen oder Polycarbonat problemlos einsetzbar.

Hinsichtlich der Auswahl geeigneter ionischer Flüssigkeiten, die bei Körpertemperatur eines Menschen oder Säugetiers einen flüssigen oder gelartigen Zustand haben, ist vorgesehen, dass die die Basis des Elektrolyten bildende ionische Flüssigkeit alkylierte Imidazolium-, Pyriginium-, Amonium- oder Phosphonium-lonen als Kationen aufweist. Im Kontext der Erfindung sind übliche Anoden- und Kathodenmaterialien gebräuchlicher elektrochemischer Elemente - insbesondere einer Lithiumbatterie - einsetzbar. Insofern ist also vorgesehen, dass das elektrochemische Element als Kathodenmaterial mindestens ein Material aus der Gruppe Mangandioxid, Kupferoxiphosphat, Lithium-Eisen-Phosphat, Lithium-Kobaltoxid, Lithium-Nickel-Kobaltoxid, Carbonmonofluorid, Silber-Vanadium-Oxid und Kupferoxid und/oder als Anodenmaterial ein Material aus der Gruppe Lithium, Kohlenstoff und Lithium-Titanoxid aufweist.

Vorteile und Zweckmäßigkeiten der Erfindung ergeben sich im übrigen aus der nachfolgenden Beschreibung eines Ausführungsbeispiels anhand der Figuren. Von diesen zeigen:
- Fig. 1A und 1B eine: Draufsicht bzw. Seitenansicht einer Kathode der Lithiumbatterie eines Gerätes gemäß einem Ausführungsbeispiel der Erfindung,
- Fig. 2: eine Seitenansicht der vollständigen Elektrodenanordnung der Lithium- batterie nach Fig. 1,
- Fig. 3A und 3B: eine schematische Draufsicht bzw. Seitenansicht der kompletten Li- thiumbatterie gemäß diesem Ausführungsbeispiel sowie
- Fig. 4: eine Prinzipskizze (als perspektivische Darstellung) eines Herzschritt- machers als Ausführungsbeispiel des erfindungsgemäßen Gerätes.

Gemäß dem Ausführungsbeispiel werden 91 % Manganoxid mit 7% expandierten Graphits und 2% eines PTFE-Pulvers homogen vermischt. Diese Mischung wird zu einer ca. 3,5 mm dicken Elektrode 1 mit einem mittig angeordneten Ableitungsgitter 2 verpresst.

Das Ableitungsgitter ist mit einem Kontaktfähnchen 3 versehen, mit dessen Hilfe die Kathode an eine Gehäusedurchführung kontaktiert werden kann. Die Gesamtoberfläche der Elektrode beträgt ca. 10 cm². Die Elektrode wird bei einer Temperatur von 270°C - 300°C und einem Druck von 10⁻³ mbar für min 1 h getrocknet.

Anschließend wird die Elektrode beidseitig mit formgleichen Lithium-Anoden 4a und 4b versehen. Diese besitzen ebenfalls Kontaktfähnchen 5a bzw. 5b, mit denen die Anoden an eine Gehäusedurchführung 5c kontaktiert werden können. Als Separator 6a und 6b dienen mikroporöse Membranen aus einem Polyolefin oder einem keramischen Material.

Dieser Aufbau wird in ein Batteriegehäuse 8 aus Kunststoff, z.B. aus Polyethylen oder Polycarbonat mit einer Wandstärke von 0,2 mm eingeschweißt. Die Elektroden der Batterie werden durch in den Kunststoff-Deckeln eingeschmolzene Kontaktstifte 9 nach außen geführt, womit sich insgesamt eine Lithiumbatterie 10 ergibt.

Anschließend wird die Batterie mit einer 0,7 M Lösung von Lithiumperchlorat in Triethylsulfoniumbis(trifluoromethylsulfonyl)mid befüllt und anschließend verschweißt.

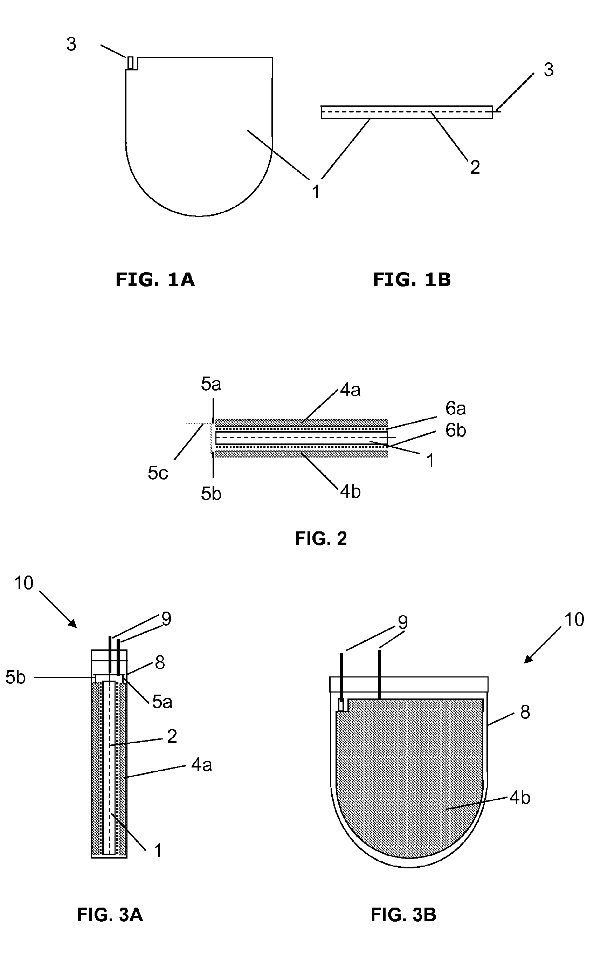

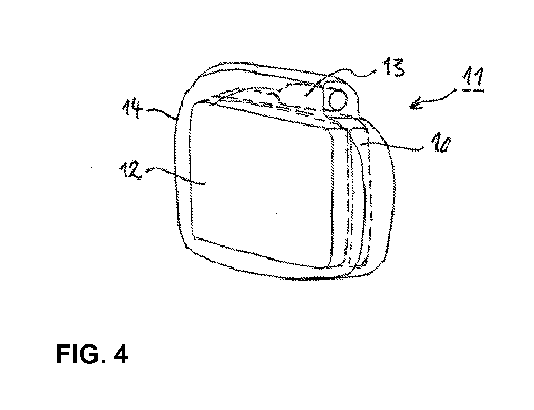

Fig. 4 zeigt schematisch einen Herzschrittmacher 11 als Ausführungsbeispiel eines erfindungsgemäßen Gerätes, welcher neben der in Fig. 3A und 3B gezeigten Lithiumbatterie 10 einen von dieser mit Strom versorgten Elektronik-Block 12 und einen mit jener verbundenen Elektrodenleitungs-Konnektor 13 in einem gasdicht verschweißten Titangehäuse 14 aufweist.

Die Ausführung der Erfindung ist nicht auf dieses Beispiel beschränkt, sondern ebenso in einer Vielzahl von Abwandlungen möglich, die im Rahmen fachgemäßen Handelns liegen.

## Patentansprüche

1. Batteriebetriebenes Gerät (11), mit einer elektrisch betriebenen Funktionseinheit (12) und einer elektrochemischen Spannungsquelle (10), die zusammen in einem im wesentlichen gasdichten Gerätegehäuse (14) untergebracht sind, wobei die elektrochemische Spannungsquelle einen Elektrolyten auf Basis einer ionischen Flüssigkeit und ein beschichtungsfreies Kunststoff-Batteriegehäuse (8) aufweist.

2. Gerät nach Anspruch 1, wobei der Elektrolyt in einem Grade frei von Lösungsmitteln und das Kunststoffgehäuse (8) derart ausgebildet sind, dass bei Betriebstemperatur kein messbares Ausgasen aus dem Batteriegehäuse auftritt.

3. Gerät nach Anspruch 1 oder 2, ausgebildet als implantierbares medizinelektronisches Gerät, insbesondere Herzschrittmacher, Defibrillator, Neurostimulator, Insulinoder Medikamentenpumpe oder dergleichen, mit gasdicht verschweißtem oder versiegeltem Metall-Gerätegehäuse.

4. Gerät nach einem der vorangehenden Ansprüche, wobei das elektrochemische Element (10) als Lithiumbatterie ausgebildet ist.

5. Gerät nach einem der vorangehenden Ansprüche, wobei das Batteriegehäuse (8) ein Spritzgussteil oder ein Folien-Tiefziehteil aufweist.

6. Gerät nach einem der vorangehenden Ansprüche, wobei das elektrochemische Element (10) einen flüssigen Elektrolyten und ein flüssigkeitsdicht ausgebildetes Batteriegehäuse (8), insbesondere mit aufgeschweißtem Deckel, aufweist.

7. Gerät nach einem der Ansprüche 1 bis 5, wobei das elektrochemische Element (10) einen gelförmigen Elektrolyten und ein nicht flüssigkeitsdicht ausgebildetes Batteriegehäuse (8), insbesondere mit formschlüssig eingesetztem Deckel, aufweist.

8. Gerät nach einem der vorangehenden Ansprüche, wobei das Batteriegehäuse (8) aus Polyethylen oder Polycarbonat ausgeführt ist.

9. Gerät nach einem der vorangehenden Ansprüche, wobei die die Basis des Elektrolyten bildende ionische Flüssigkeit alkylierte Imidazolium-, Pyridinium-, Ammoniumoder Phosphonium-lonen als Kationen aufweist.

10. Gerät nach einem der vorangehenden Ansprüche, wobei das elektrochemische Element (10) als Kathodenmaterial (1) mindestens ein Material aus der Gruppe Mangandioxid, Kupferoxiphosphat, Lithium-Eisen-Phosphat, Lithium-Kobaltoxid, Lithium-Nickel-Kobaltoxid, Carbonmonofluorid, Silber-Vanadium-Oxid und Kupferoxid und/oder als Anodenmaterial (4a, 4b) ein Material aus der Gruppe Lithium, Kohlenstoff und Lithium-Titanoxid aufweist.
